(19)

Europäisches
Patentamt
European
Patent Office
Office européen
des brevets

(11) **EP 2 789 359 A2**

(12) **EUROPEAN PATENT APPLICATION**

(43) Date of publication:
**15.10.2014 Bulletin 2014/42**

(21) Application number: **14176792.1**

(22) Date of filing: **30.08.2007**

(51) Int Cl.:
*A61M 16/00* ^(2006.01)   *A61M 16/06* ^(2006.01)

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **30.08.2006  US 823934 P**

(62) Document number(s) of the earlier application(s) in accordance with Art. 76 EPC:
**07800212.8 / 2 063 941**

(71) Applicant: **ResMed Ltd.**
**Bella Vista, New South Wales 2153 (AU)**

(72) Inventor: **The designation of the inventor has not yet been filed**

(74) Representative: **Vossius & Partner**
**Siebertstrasse 4**
**81675 München (DE)**

Remarks:
This application was filed on 11-07-2014 as a divisional application to the application mentioned under INID code 62.

(54) **Determination of leak during CPAP treatment**

(57)    A ventilatory support apparatus, the apparatus being configured to:determine an index indicative of an extent to which non-deliberate leak is uncompensated; and adjust a level of ventilatory support in accordance with said index.

Printed by Jouve, 75001 PARIS (FR)

## Description

### Related Applications

This application claims priority of US Provisional Application 60/823,934, filed August 30, 2006, the specification and drawings of which are incorporated herein by reference.

### Field Of The Invention

This invention relates to treatment of apneas and other respiratory disorders. In particular it relates to methods and apparatus for the determination of leakage airflow and true respiratory airflow, during the mechanical application of positive airway pressure.

### Background of the Invention

For the treatment of apneas and other respiratory disorders occurring during sleep, breathable gas is supplied from a mechanical respirator or ventilator, for example via a mask, at a pressure which may be higher during inspiration and lower during expiration. (In this specification any reference to a "mask" is to be understood as including all forms of devices for passing breathable gas to a person's airway, including nose masks, nose and mouth masks, nasal prongs/pillows and endotracheal or tracheostomy tubes. The term "ventilator" is used to describe any device that does part of the work of breathing.) Typically one measures the subject's respiratory airflow during mechanical ventilation to assess adequacy of treatment, or to control the operation of the ventilator.

Respiratory airflow is commonly measured with a pneumotachograph placed in the gas delivery path between the mask and the pressure source. Leaks between the mask and the subject are unavoidable. The pneumotachograph measures the sum of the respiratory airflow plus the flow through the leak. If the instantaneous flow through the leak is known, the respiratory airflow can he calculated by subtracting the flow through the leak from the flow at the pneumotach.

Some methods to correct for the flow through the leak assume (i) that the leak is substantially constant, and (ii) that over a sufficiently long time, inspiratory and expiratory respiratory airflow will cancel. If these assumptions are met, the average flow through the pneumotach over a sufficiently long period will equal the magnitude of the leak, and the true respiratory airflow may then be calculated as described.

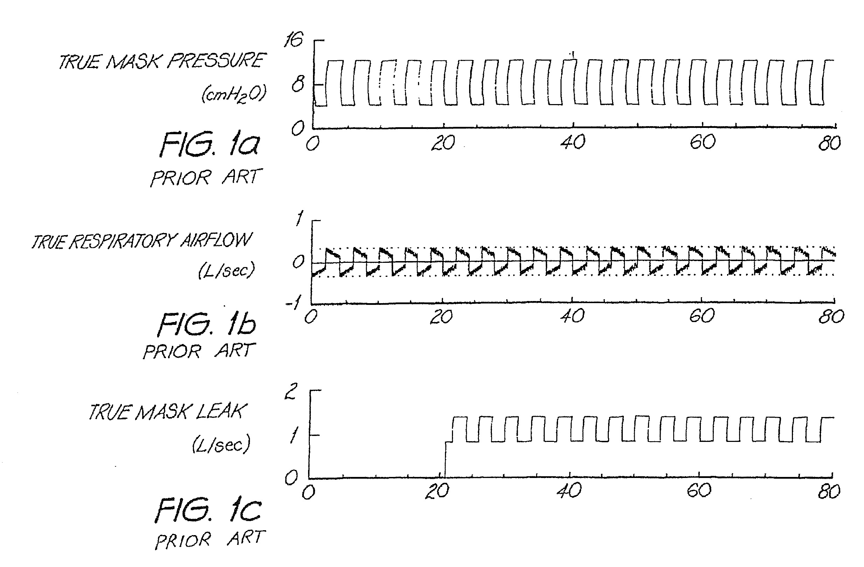

The known method is only correct if the pressure at the mask is constant. If, on the other hand, the mask pressure varies with time (for example, in the case of a ventilator), assumption (i) above will be invalid, and the calculated respiratory airflow will therefore be incorrect. This is shown markedly in FIGS. 1a-1f.

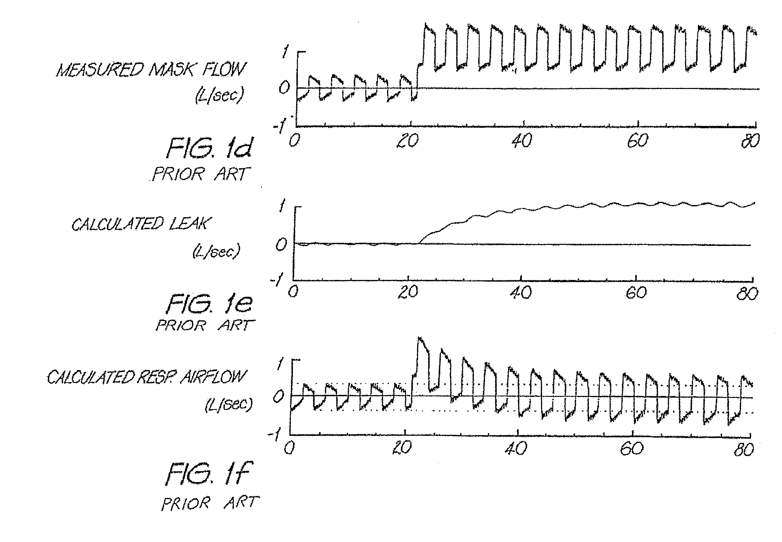

FIG. 1 a shows a trace of measured mask pressure in bi-level CPAP (Continuous Positive Airway Pressure) treatment between about 4 cm $H_2O$ on expiration and 12 cm $H_2O$ on inspiration. FIG. 1b shows a trace of true respiratory airflow in synchronism with the mask pressures. At time=21 seconds a mask leak occurs, resulting in a leakage flow from the leak that is a function of the treatment pressure, as shown in FIG. 1c. The measured mask flow shown in FIG. 1d now includes an offset due to the leak flow. The prior art method then determines the calculated leak flow over a number of breaths, as shown in FIG. 1e. The resulting calculated respiratory flow, as the measured flow minus the calculating leak flow is shown in FIG. 1f, having returned to the correct mean value, however is incorrectly scaled in magnitude, giving a false indication of peak positive and negative airflow.

Another prior art arrangement is disclosed in European Publication No. 0 714 670 A2, including a calculation of a pressure-dependent leak component. The methodology relies on knowing precisely the occurrence of the start of an inspiratory event and the start of the next inspiratory event. In other words, the leak calculation is formed as an average over a known breath and applied to a subsequent breath.

This method cannot be used if the moment of start and end of the previous breath are unknown. In general, it can be difficult to accurately calculate the time of start of a breath. This is particularly the case immediately following a sudden change in the leak.

Furthermore, the method will not work in the case of a subject who is making no respiratory efforts, and is momentarily not being ventilated at all, for example during an apnea, because for the duration of the apnea there is no start or end of breath over which to make a calculation.

In US patent 6,152,129 (Berthon-Jones) the leak is determined by first estimating the conductance of the leak path from the longterm orifice flow:

$$\frac{1}{R_L} = \frac{<Q>}{<\sqrt{p}>},$$

where $G_L = 1/R_L$ is conductance (L denotes leak), Q is instantaneous flow, p is instantaneous pressure and <> denotes a longterm average calculated for example by low pass filtering with an IIF or other filter having a long time constant. Note that the word "average" as used herein contains the general sense inclusive of the result of a low pass filtering step, and is not limited to an arithmetic mean or other standard average such as the RMS average.

The instantaneous leak flow, based on the model of the flow through an orifice is then $Q_L = \frac{1}{R_L}\sqrt{p}$ .

Note that the instantaneous respiratory airflow is then QR = Q - Q$_L$.

Berthon-Jones attempts to deal with sudden changes in instantaneous leak flow by dynamically adjusting the filter's time constant using fuzzy logic, lengthening the time constant if it is certain that the leak is steady, reducing the time constant if it is certain that the leak has suddenly changed, and using intermediately longer or shorter time constants if it is intermediately certain that the leak is steady.

Berthon-Jones also develops a jamming index by fuzzy logic to deal with the case of a large and sudden increase in the conductance of the leak, in which case the calculated respiratory airflow will be incorrect. In particular during apparent inspiration, the calculated respiratory airflow will be large positive for a time that is large compared with the expected duration of a normal inspiration. Conversely, if there is a sudden decrease in conductance of the leak, then during apparent expiration the calculated respiratory airflow will be large negative for a time that is large compared with the duration of normal expiration.

Therefore, the jamming index, i.e. an index of the degree of certainty that the leak has suddenly changed, is derived, such that the longer the airflow has been away from zero, and by a larger amount, the larger the index. The explicit calculation of the jamming index by fuzzy logic is described in the '129 patent, which is incorporated herein by reference.

The time constant for the low pass filters is then adjusted to vary inversely with the jamming index. In operation, if there is a sudden and large change in the leak, the index will be large, and the time constant for the calculation of the conductance of the leak will be small, allowing rapid convergence on the new value of the leakage conductance. Conversely, if the leak is steady for a long time, the index will be small, and the time constant for calculation of the leakage conductance will be large, enabling accurate calculation of the instantaneous respiratory airflow. In the spectrum of intermediate situations, where the calculated instantaneous respiratory airflow is larger and for longer periods, the index will be progressively larger, and the time constant for the calculation of the leak will progressively reduce. For example, at a moment in time where it is uncertain whether the leak is in fact constant, and the subject merely commenced a large sigh, or whether in fact there has been a sudden increase in the leak, the index will be of an intermediate value, and the time constant for calculation of the impedance of the leak will also be of an intermediate value.

## Brief Description of the Invention

This invention rapidly determines the instantaneous leak in a CPAP system without detailed modeling the source of the leak and without having to determine the precise phase in a breathing cycle at which the leak occurs. It relies instead on the use of timers to define the breathing cycle and a calculation to assure that the instantaneous flow is compared to the flow over a time period long enough to include an entire breath. It does this by looking backward to include an entire phase cycle. This avoids having to take long term averages over multiple breaths, or to have a model that recognized the beginning and end of a breath.

Sudden changes in a leak are recognized and expressed as a jamming index value, which is then used as a parameter to control the temporary secession of updating flow values.

Further forms of the invention include those set out in the claims.

Further aspects of the invention are described as follows:

1. A method for determining the amount of leak in a CPAP system controlled by timers, comprising determining the length of a time interval at least as long as a single breath, integrating airflow over a preceding time interval as long as said time interval, omitting said integration over said interval when a jamming index indicates that the leak has suddenly changed, until a measure of leak variation ceases to indicate a rapid rate of change, determining the amount of leak as a function of said integration.

2. A method for determining the airflow in a CPAP system controlled by timers, comprising setting cycling thresholds that vary with time, determining a breath phase from the cycling thresholds, looking backwards in time for a phase equal to the current phase, determining the length of a time interval at least as long as that between equal phase points, integrating airflow over a preceding time interval as long as said time interval.

3. The method for determining the airflow in a CPAP system controlled by timers of aspect 2, further comprising omitting said integration over said interval when a jamming index indicates that the leak has suddenly increased, until a measure of leak variation ceases to indicate a rapid rate of change, correcting said airflow by the amount of leak as a function of said integration.

4. A CPAP system controlled by timers, comprising a controller for determining the length of a time interval at least as long as a single breath, integrating airflow over a preceding time interval as long as said time interval, omitting said integration over said interval when a jamming index indicates that the leak has suddenly increased, until a measure of leak variation ceases to indicate a rapid rate of change, determining the amount of leak as a function of said integration.

5. A CPAP system controlled by timers, comprising a controller for
setting cycling thresholds that vary with time, determining breath phases from the cycling thresholds,
looking backwards in time for a phase equal to the current phase,
determining the length of a time interval at least as long as that between equal phase points,
integrating airflow over a preceding time interval as long as said time interval.

6. The CPAP system controlled by timers of aspect 5, further comprising
omitting said integration over said interval when a jamming index indicates that the leak has suddenly increased, until a measure of leak variation ceases to indicate a rapid rate of change,
correcting said airflow by the amount of leak as a function of said integration.

7. The CPAP system controlled by timers of aspect 5, in which, when jamming is present suspends changes in pressure support.

8. A method of determining an air leak flow rate from a respiratory device connected to a patient breathing in breathing cycles, the method comprising the steps of:

(i) measuring a flow rate of air from the respiratory device;
(ii) determining a duration of a breathing cycle;
(iii) calculating an average of the flow rate of air over a period at least equal to said duration;
(iv) determining the air leak flow rate to be the difference between said air flow rate and said average.

9. The method of aspect 8 wherein said flow rate of air is measured continuously.

10. The method of aspect 8 wherein said determining of a duration of a breathing cycle is determined continuously.

11. The method of aspect 8 wherein said calculating an average of the flow rate of air is calculated continuously.

12. The method of aspect 8 wherein said calculating an average of the flow rate of air is calculated from a mid-inspiratory point of a first breathing cycle to a mid-inspiratory point of a second breathing cycle.

13. The method of aspect 8 wherein said calculating an average of the flow rate of air is calculated from a mid-expiratory point of a first breathing cycle to a mid-expiratory point of a second breathing cycle.

14. The method of aspect 12 or 13 wherein said second breathing cycle is adjacent said first breathing cycle.

15. The method of aspect 8 further comprising the step of determining whether the air leak flow rate is rapidly changing.

16. The method of aspect 8 without further comprising the step of determining a transition point from inspiration to expiration.

17. The method of aspect 8 without further comprising the step of determining a transition point from expiration to inspiration.

18. A method of determining an air leak flow rate at a first point in time from a respiratory device connected to a patient breathing in breathing cycles, the method comprising the steps of:

(i) determining a flow rate of air from the respiratory device;
(ii) determining a phase function of the breathing cycle the patient;
(iii) determining a duration of the breathing cycle;
(iv) selecting a second point in said function of phase corresponding to the first point in time in a previous breath;
(v) integrating the flow rate between said first and second points;
(vi) determining said leak flow rate by comparing said flow rate of air and the result of said integration.

19. The method of aspect 18 wherein said phase function is a discontinuous function.

20. A method of determining an air leak flow rate at a first point in time from a respiratory device connected to a patient breathing in breathing cycles comprising the steps of:

(i) determining a flow rate of air at said first time point;
(ii) determining a first phase value in the respiration cycle of the patient corresponding to said first point in time;
(iii) determining a second point in a prior respiration cycle when the respiration was at approximately the same phase value as said first phase value;

(iv) calculating an average flow rate over the interval between said first and second points;

(v) determining the leak flow by comparing said flow rate and said average flow rate.

21. A method of providing pressure support to a patient comprising the steps of:

(i) determining a leak flow rate;

(ii) determining whether a leak flow rate is rapidly changing;

(iii) determining a level of pressure support;

(iv) providing said level of pressure support to the patient;

(v) maintaining substantially the same level of pressure support if the leak flow rate is rapidly changing.

22. A respiratory device for connection to a patient breathing in cycles, including a controller for:

(i) measuring a flow rate of air from the respiratory device;

(ii) determining a duration of a breathing cycle;

(iii) calculating an average of the flow rate of air over a period at least equal to said duration;

(iv) determining the air leak flow rate to be the difference between said air flow rate and said average.

23. A respiratory device for connection to a patient breathing in cycles, including a controller for determining an air leak flow rate at a first point in time from the respiratory device by

(i) determining a flow rate of air from the respiratory device;

(ii) determining a phase function of the breathing cycle the patient;

(iii) determining a duration of the breathing cycle;

(iv) selecting a second point in said function of phase corresponding to the first point in time in a previous breath;

(v) integrating the flow rate between said first and second points; and

(vi) determining said leak flow rate by comparing said flow rate of air and the result of said integration.

24. A respiratory device for connection to a patient breathing in cycles, including a controller for determining an air leak flow rate at a first point in time from the respiratory device by:

(i) determining a flow rate of air at said first time point;

(ii) determining a first phase value in the respiration cycle of the patient corresponding to said first point in time;

(iii) determining a second point in a prior respiration cycle when the respiration was at approximately the same phase value as said first phase value;

(iv) calculating an average flow rate over the interval between said first and second points; and

(v) determining the leak flow by comparing said flow rate and said average flow rate.

25. Apparatus for providing pressure support to a patient, comprising a controller for:

(i) determining a leak flow rate;

(ii) determining whether a leak flow rate is rapidly changing;

(iii) determining a level of pressure support;

(iv) providing said level of pressure support to the patient;

(v) maintaining substantially the same level of pressure support if the leak flow rate is rapidly changing.

## Detailed Description of Preferred Embodiments of the

## Invention

The present invention is motivated by the desire to determine instantaneous leak without having to resort to the computationally expensive processes of fuzzy logic. In particular it provides a system in which a patient's breathing effort is synchronized with the flow from a ventilator by the use of timers rather than by a complex measurement of the phase of a breathing cycle. Synchronization is flow triggered and phase is a bookkeeping device only. Cycling between pressures suitable for inspiration and expiration is controlled by thresholds that change during breaths.

What is important is achieving synchronization between the patient's breathing effort and the action of the ventilator. A target ventilation is defined and supported by a variable backup rate. Servo control of gross alveolar ventilation is provided in which the further a breathing pattern moves from the target, the faster the response of the system. This is designed to stop small breaths from counting for effective ventilation.

One problem is caused by sudden changes in a leak. It is necessary to respond as quickly as a monitored parameter recognizes the change. This is accomplished by having controlling thresholds respond to jamming.

The present invention uses triggering to achieve synchronization between the patient's effort and the ventilator functions. This is accomplished by setting cycling thresholds that vary with time. The parameters are $T_i$Min, $T_i$Max, $T_e$Min and $T_e$Max, respectively the min-

imum and maximum time that inspiration and expiration can persist. Triggering thus consists in the setting of $T_iMin$, $T_iMax$, $T_eMin$ and $T_eMax$.

These relate to instantaneous backup rates, since

$$T_{tot}Max = TiMax + TeMax. = 1/Backup\text{-}rate.$$

Triggering of a change in flow to begin an inspiration-expiration cycle is subject to an adjustable threshold value. However there can be no triggering before the minimum expiration time (TeMin). Cycling from inspiration to expiration is subject to an initial absolute refractory period (TiMin), where the threshold is an adjustable proportion of the peak flow, but the threshold is constrained to be within predetermined limits. It is therefore less likely to cycle early in timed breaths (i.e. relative refractory period) in order to make too-short timed breaths less likely. In any event, cycling will occur when a maximum inspiratory time is reached, which time is calculated automatically.

Typically, a clinician sets the backup rate to be delivered during a sustained period of timed breaths ("apnoeic backup rate"). This can be set at the patient's optimum rate at end of learning period. The backup rate during spontaneous breathing (triggered breaths) is 2/3 of apnoeic backup rate. On a transition from triggered to timed breaths, the backup rate increases progressively, typically over 5 breaths, to the apnoeic rate. The backup rate increases faster if ventilation well below target, more slowly if at target, not at all if well above target. Any triggered breath causes the backup rate to drop to 2/3 of apnoeic rate.

**Phase as a measure of Cycle Progress**

The breath phase, is by definition 0 at the start of inspiration, 0.5 at the start of expiration, and approaches 1 at the end of expiration. As a bookkeeping device it is sufficient to consider the phase as a linear function between transition points. During inspiration the phase may be linearized in segments at such a rate of change during inspiration that the phase will reach 0.5 at TiMax and at such a rate of change of phase during expiration that phase will reach 1 at TeMax.

The phase will thus show jumps if the switch from inspiration to expiration occurs as the result of a timeout. Thus unlike the situation in Berthon-Jones, where the phase is forced to follow a smooth curve, the phase now is a broken series of line segments exhibiting jumps. There are a few cases to be considered: If TiMax is reached, cycling occurs due to time and the phase curve is continuous. If cycling from inspiration to expiration occurred due to a flow threshold, then the phase will have an upward jump. If $T_eMax$ is reached then triggering occurs due to time, a backup breath is delivered and the phase drops suddenly to zero. If triggering occurs due to flow to end the expiration then the phase will not reach 1.

**Correcting for Leak**

Leak makes the estimation of patient flow difficult., since patient flow measurement is the basis of a ventilation estimate. What is provided is a leak algorithm that responds rapidly to changes in leak, in a few breaths yet has very stable baseline, which effectively suspends changes in pressure support while adapting to a new leak level after a big change, and can handle moderately large leaks (0.6 l/s).

To determine leak one can integrate the flow over a time period as long as a single breath. So that this can be accomplished immediately, the invention looks back from the present time to a time when the respiration was at the same phase as the phase at the current time. Starting with the current phase, say $\varphi_0$, the invention looks backwards in time for the most recent phase in the interval $[\varphi_0 - 0.75, \varphi_0 - 0.25]$. This is seeking a point in time at least 0.25 of a breath before the present. When such a phase is found, the invention calculates ($\varphi_1 = \varphi_0 - 0.25$ and looks backward for a phase in he interval $[\varphi_1 - 0.75, \varphi_1 - 0.25]$. This is continued, 0.25 at a time, i.e. $\varphi_{l+1} = \varphi_l - .025$. When a phase is found which is in $[\varphi_3 - .075, \varphi_3 - 0.25]$ the iteration ceases, since this is just $[\varphi_0 - 0.5, \varphi_0]$. If phase varied continuously this would have found exactly $\varphi_0$; in reality it will most likely find $\varphi_0 - \varepsilon$, where hopefully $\varepsilon$ is small. By proceeding in this manner we have some confidence that the phase has gone backward rather than forward. This algorithm will regard two phase transitions of 0.5 in succession as being movement backward, thought the actual direction is of course actually indeterminate. If this algorithm fails to find a point between the present moment and a time $T_{leak}$ before the present which meats this criterion, we take the averaging period to be $T_{leak}$. As an implementation detail, to reduce computational requirements, the leak flow values may be averaged over the last 5 points (0.1 seconds) and stored in a buffer accompanied by the associated breath phase, so that the search for the last breath was in a buffer of 100 points, and done every 0.1 seconds. The 50 Hz averaged leak estimate can then be calculated at 50 Hz by linear interpolation between the most recent averaged leak estimate and the averaged leak estimate just before it.

**Setting the Backup and Timing Parameters**

The backup rate varies from breath to breath, so the slope of the phase curves may vary from breath to breath: At each breath the backup period is calculated ( = 60 seconds / backup rate in breaths/min). TiMax is calculated at the start of each inspiration as the backup period multiplied by the inspiratory fraction, so the slope of the inspiratory breath phase line ( = 0.5 / TiMax) varies from breath to breath. TeMax is calculated at the start of each expiration to be that time which will deliver the current backup rate ( = backup period - actual inspiratory time), so the slope of the expiratory breath phase line ( = 0.5 / TeMax) varies from breath to breath.

The servoventilation of the present invention automatically adjusts pressure support to provide at least a target ventilation, between a minimum pressure support (which may be zero) and a maximum pressure support. Min and max pressure support are set by a clinician. The target ventilation is the non-anatomical-deadspace ventilation (a crude approximation to alveolar ventilation). The aim is mainly to prevent small breaths at a high rate from being counted as effective ventilation. The anatomical deadspace is set manually.

**Clinical Procedure**

The clinical procedure is to learn the target ventilation and backup rate while the patient is awake. Then a target ventilation ramp and EPAP is set. Then possibly adjust the maximum inspiratory fraction and the cycling threshold, which is a proportion of the peak flow. A default value is 25%.

**Jamming**

Jamming is a state in which there exists a change in a leak that has not been fully compensated for. The presence of a leak can change the flow so that it doesn't get close to its zero crossing points at which the transition from inspiration to expiration is usually recognized. Thus if the pressure is adjusted to switch from inspiration to expiration at such a zero crossing point, the change will not be recognized and the pressure will remained "jammed" in one phase. However the low pass filtered flow should oscillate around a base line that can be recognized as the new criteria for the phase change.

The jamming index J is related to leak flow by the formula:

$$Jk_J + (1 - J)k_S \sqrt{p_{mask}} = Q_{leak} ,$$

where J=0 indicates a stable leak through some orifice and J=1 a sudden leak.

When jamming is observed, i.e. a rapid change of leak, the system suspends changes in pressure support. Otherwise the pressure support is adjusted automatically.

**Claims**

1. A ventilatory support apparatus, the apparatus being configured to:

    determine an index indicative of an extent to which non-deliberate leak is uncompensated; and
adjust a level of ventilatory support in accordance with said index.

2. The apparatus of claim 1, wherein the index is a jamming index, preferably resulting from a rapid change in leak.

3. A ventilator support apparatus according to claim 1 or 2,
wherein the level of ventilatory support is adjusted at a rate which is a function of said index.

4. The apparatus according to any one of the preceding claims, wherein said rate of adjustment is decreased with increasing uncompensated non-deliberate leak.

5. The apparatus according to any one of the preceding claims, further comprising a servo ventilator configured to adjust ventilator support.

6. The apparatus according claim 5, wherein the servo ventilator is adapted to slow down or suspens changes in the pressure support.

7. The apparatus according claim 5 or 6, wherein the servoventilor controller adjusts ventilatory support by:

    determining an index value based on a change in an uncompensated leak level associated with the ventilatory support apparatus; modifying a pressure support if the index value is determined to be less than a threshold value.

8. The apparatus according to any one of claims 5 to 7, comprising modifying the pressure support if the threshold value is between 0 and 1.

9. The apparatus according to any one of claims 5 to 8, further comprising calculating a change in respiratory support associated with the ventilatory support apparatus and modifying the pressure support with an amount related to a predetermined change in pressure support if the index value is less than or equal to 0.1.

10. The apparatus according to any one of claims 5 to 9, further comprising calculating a change in respiratory support associated with the ventilatory support apparatus and not modifying the pressure support if the index value is greater than or equal to 0.3.

11. The apparatus according to any one of the preceding claims, wherein the index is related to a mask leak conductance through the equation

$$G_j = JG_1 + (1-J)G_2.$$

wherein:

$G_j$ is an estimate of mask leak conductance,
$G_1$ is the first estimate of leak conductance,
$G_2$ is the second estimate of leak conductance, the second estimate changing slower and being more stable than the first estimate, and
$J$ is the index of the uncompensated mask leak.

12. The apparatus according to claim 11, wherein a non-deliberate mask leak is determined using an estimate of the mask leak conductance and mask pressure.

EP 2 789 359 A2

TRUE MASK PRESSURE

(cmH$_2$O)

**FIG. 1a**
PRIOR ART

TRUE RESPIRATORY AIRFLOW

(L/sec)

**FIG. 1b**
PRIOR ART

TRUE MASK LEAK

(L/sec)

**FIG. 1c**
PRIOR ART

MEASURED MASK FLOW
(L/sec)

FIG. 1d
PRIOR ART

CALCULATED LEAK
(L/sec)

FIG. 1e
PRIOR ART

CALCULATED RESP. AIRFLOW
(L/sec)

FIG. 1f
PRIOR ART

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 60823934 B
- EP 0714670 A2

- US 6152129 A, Berthon-Jones